# EUROPEAN PATENT SPECIFICATION

(11) **EP 4 019 421 B1**
(45) Date of publication and mention of the grant of the patent: **22.05.2024**
(21) Application number: 20306676.6
(22) Date of filing: 23.12.2020
(51) Int. Cl.: B65D 21/02, A61J 1/16, A61M 5/00

(54) **A SPACER STRUCTURE FOR SEPARATING TWO STACKED TUBS CONFIGURED FOR THE STORAGE AND/OR TRANSPORT OF A PLURALITY OF MEDICAL CONTAINERS, AND A PACKAGING INCLUDING SAID SPACER**
ABSTANDSHALTERSTRUKTUR ZUM TRENNEN VON ZWEI GESTAPELTEN KISTEN, DIE ZUM AUFBEWAHREN UND/ODER TRANSPORTIEREN EINER VIELZAHL VON MEDIZINISCHEN BEHÄLTERN KONFIGURIERT SIND, UND EINE VERPACKUNG MIT BESAGTEM ABSTANDHALTER
STRUCTURE D'ENTRETOISE POUR SÉPARER DEUX BACS EMPILÉS CONFIGURÉS POUR LE STOCKAGE ET/OU LE TRANSPORT D'UNE PLURALITÉ DE CONTENEURS MÉDICAUX, ET EMBALLAGE COMPRENANT LADITE ENTRETOISE

(43) Date of publication of application: 29.06.2022
(73) Proprietor: Becton Dickinson France, 38800 Le Pont-de-Claix (FR)
(72) Inventor: EYMERY, Anaïs, 38450 SAINT-GEORGES-DE-COMMIERS (FR); LE LOC'H, Clémentine, 38240 Meylan (FR)
(74) Representative: Germain Maureau

(56) References cited:
- WO-A1-2009/053434
- DE-U1- 9 318 800
- US-A1- 2015 166 243

## Description

The present invention relates to a packaging including a second tub stacked onto a first tub along a central vertical axis A, and a spacer structure arranged between the first tub and the second tub stacked onto each other along the central vertical axis A, said first tub and said second tub being configured for the transport or storage of a plurality of medical containers such as syringes.

Medical containers such as pre-fillable or prefilled syringes are usually supported by a plate-shaped tray. This plate-shaped tray, also called a "nest", is placed in a box-shaped housing protecting the medical containers during storage or transport.

This box-shaped housing, also called a "tub", usually defines an opening for insertion or removal of the nest. The insertion or removal movement of the nest relative to the tub typically takes place along a vertical axis A. A cover film, such as a Tyvek^{®} sheet, seals the opening in order to keep sterility inside the tub and to maintain the medical containers inside the tub. This cover film is usually permeable to a sterilization gas such as ethylene oxide.

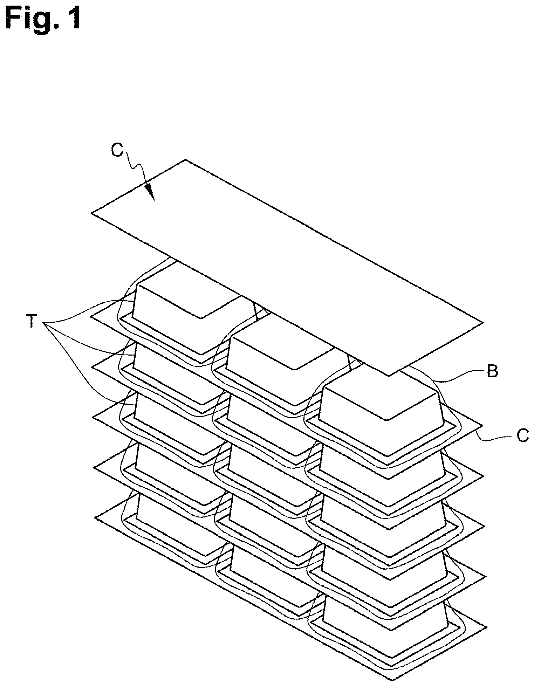

The tubs are usually disposed side by side and, as shown in Figure 1, a horizontal row of tubs T may be stacked onto another horizontal row of tubs along the vertical axis A during storage or transport. Typically, a cardboard C may be placed between two rows of tubs, and all the different tubs are disposed in a unique big box. When one wants to remove a tub from said big box, he needs to remove all the tubs in order to have access to all of them. Moreover, nothing prevents the axial and lateral movements of tubs with respects to other tubs. Lastly, when the cardboard is not present, the stacking of the tubs involves the cover film of an upper tub to rest on the bottom of a lower tub. The weight of this upper tub, and of any other tub stacked onto this upper tub, may cause damage to the cover film. There is therefore a need to protect the cover films during transport or storage of the medical containers, and to improve the handling of the tubs comprising said medical containers.

Besides, each tub is usually enclosed within an individual sealing bag, also called header bag, referenced B in the Figure 1. Header bags are disclosed for instance by the document EP2119463. This sealing bag helps maintain sterility of the medical containers contained in the tub. Said sealing bag needs to be opened for removing the tub and there is thus as many sealing bags as tubs to open in order to remove the medical containers. This involves several steps when removing the tubs from the big box. WO 2009/053434 A1 discloses a packaging including a second tub stacked onto a first tub along a central vertical axis, and a spacer structure arranged between the first tub and the second tub stacked onto each other along the central vertical axis A, said first tub and said second tub being configured for the transport or storage of a plurality of medical containers such as syringes, wherein the spacer structure comprises a central portion.

In this context, an object of the present invention is to provide a spacer structure that alleviates the above-mentioned drawbacks by allowing to protect the cover films and by preventing the axial and lateral movements of the tubs during transport.

A first aspect of the invention is a packaging including a second tub stacked onto a first tub along a central vertical axis A, and a spacer structure arranged between the first tub and the second tub stacked onto each other along the central vertical axis A, said first tub and said second tub being configured for the transport or storage of a plurality of medical containers such as syringes, wherein the spacer structure comprises:
a central portion,
supporting arms outwardly extending from said central portion, said supporting arms and said central portion being configured to be located between a cover film of the first tub and a bottom of the second tub; wherein the supporting arms comprise
first maintaining means configured to prevent radial displacements of the first tub relative to the spacer structure; and
second maintaining means configured to prevent radial displacements of the second tub relative to the spacer structure.

The spacer structure of the invention therefore allows to stack several tubs onto each other along a vertical axis A in a stable manner due to the first and second maintaining means. It is therefore possible to pack the stacked tubs into a single big bag, i.e. a collective sealing bag configured to enclose a plurality of tubs, so as to optimize the medical containers removal process. Besides, the horizontal arms interposed between the cover film of the upper tub and the bottom of the lower tub enables to distribute the load so as to protect the cover film. Meanwhile, the arm-shaped spacer structure defines large spaces between these arms that permit efficient circulation of a sterilization gas through the film cover.

In an embodiment, the supporting arms are plate-shaped.

In an embodiment, the supporting arms are distributed at regular angles around the vertical axis A.

The above features enhance the load distribution and therefore improve the film cover protection.

In an embodiment, the supporting arms form a Y-shaped, H-shaped or X-shaped spacer structure.

This provides a compromise between stability of the stacked tubs, load distribution to protect the cover film, and the material costs of the spacer structure.

In an embodiment, at least two of the supporting arms are configured to engage a same side of the first tub.

In an embodiment, the first and the second maintaining means include protrusions protruding from, respectively, a first side and an opposite second side of the supporting arms.

The protrusions may be substantially orthogonal to the supporting arms.

This allows a vertical positioning or removal of the tubs with respect to the spacer structure in order to satisfy with the ease requirement of the positioning or removal process.

In an embodiment, the protrusions are shaped to engage lateral sides or corners of, respectively, the first tub or the second tub.

In an embodiment, the spacer structure is made of a polymer material, a cellulose material such as molded cellulose, or a combination thereof.

More specifically, the polymer material may be any of polypropylene PP, polystyrene PS, polyethylene terephthalate PET, acrylonitrile-butadiene-styrene ABS, polycarbonate PC, or combinations thereof.

In an embodiment, the central portion comprises a notch or a window.

In an embodiment, the packaging includes a single collective sealing bag enclosing at least the first tub, the second tub and the spacer structure.

The invention and the advantages arising therefrom will clearly emerge from the detailed description that is given below with reference to the appended drawings as follows:
- Figure 1 is a perspective view of stacked tubs,
- Figure 2 is a perspective view of an opened and empty tub,
- Figure 3 is a perspective view of an opened tub for storage and transport of a plurality of medical containers supported by a nest;
- Figure 4 is a perspective of a spacer structure according to the invention;
- Figure 5 is a perspective view of a packaging according to an embodiment of the invention;
- Figure 6 is a perspective of a packaging according to an embodiment of the invention.

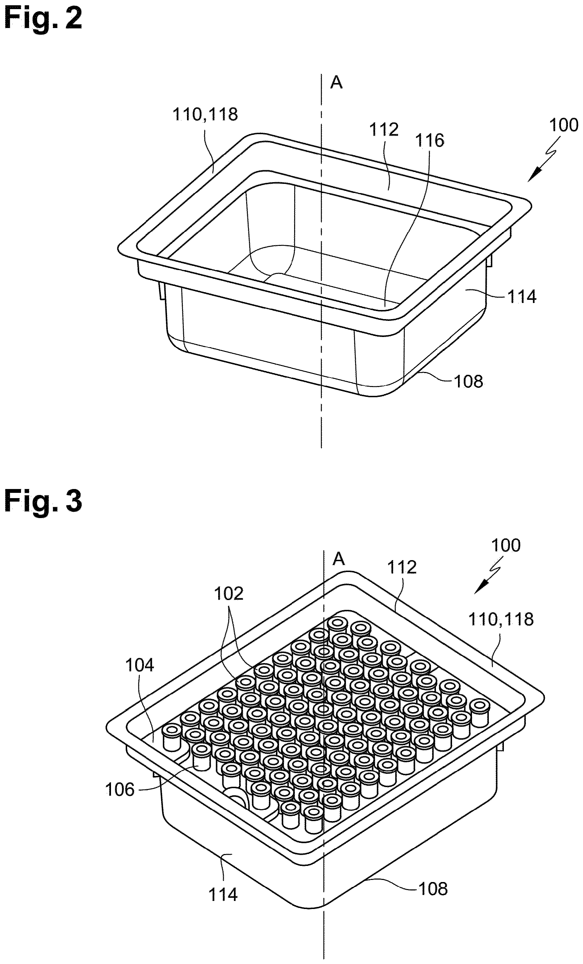

With reference to Figures 2 and 3 is shown a box-shaped housing, hereafter the tub 100, for storage and transport of a plurality of medical containers 102, such a pre-filled or prefillable syringes. The medical containers 102 are supported on a plate-shaped tray, the nest 104, that is contained inside the tub 100. The nest 104 may be inserted inside the tub 100, or removed from the tub 100, by a vertical movement along a longitudinal axis A. The medical containers 102 are maintained parallel to said vertical axis A by means of apertures and guiding elements 106 provided on the nest 104.

The tub 100 includes a bottom end 108 and an opposite upper end 110, said upper end 110 defining an opening 112. The bottom and upper ends 108, 110 may be connected by lateral walls 114. The lateral walls 114 may taper towards the bottom end 108 and said bottom end 108 may include a closed bottom wall 116. The upper end 110 may be provided with a flange 118 and the opening 112 is usually sealed by a cover film, typically a Tyvek^{®} film (not shown). The cover film is substantially orthogonal to the vertical axis A. The cover film is permeable to sterilization gases, such as ethylene oxide (EtO) and is attached, for example glued, onto the peripheral flange 118 of the tub 100. In the removal process of the nest 104 from the tub 100, the cover film is manually or automatically peeled off.

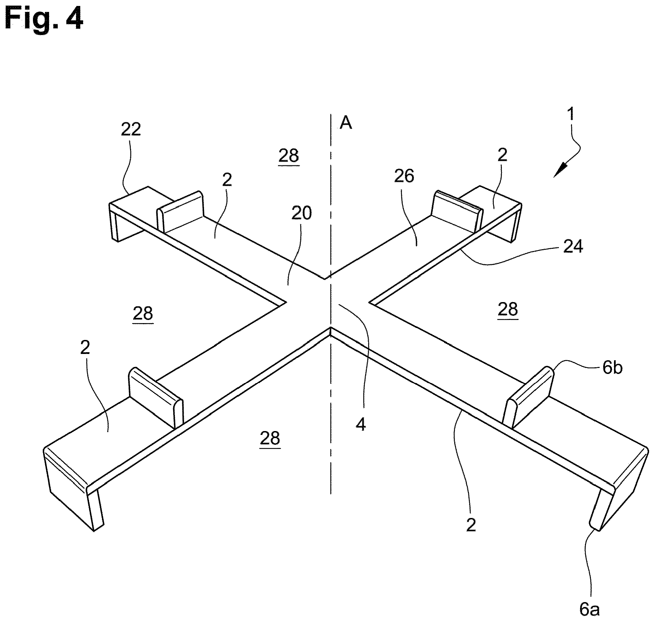

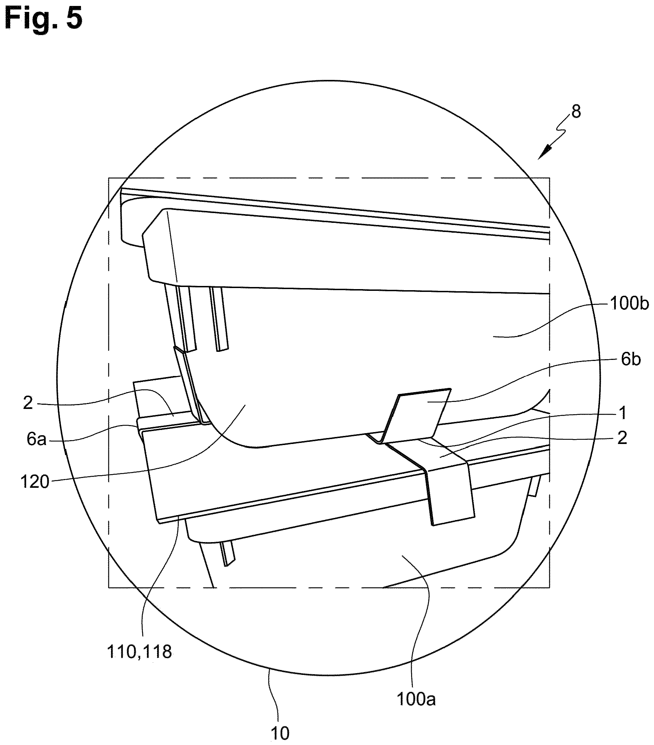

With reference to Figures 4 and 5 is shown a spacer structure 1 according to an embodiment of the invention. The spacer structure 1 is intended to separate a first and a second tubs 100a, 100b stacked onto each other so as to maintain these two tubs 100a, 100b in a stable manner with respect to the vertical axis A and to protect the cover film of one of these two tubs 100a, 100b by distributing the load onto the flange 118 of one of tub 100a, 100b. The spacer structure 1 also enables avoiding the radial movement of one of the two tubs 100a, 100b with respect to the other tub 100a, 100b. The spacer structure 1 is also intended to leave the cover film at least partially uncovered so as to allow circulation of a sterilization gas through the cover film.

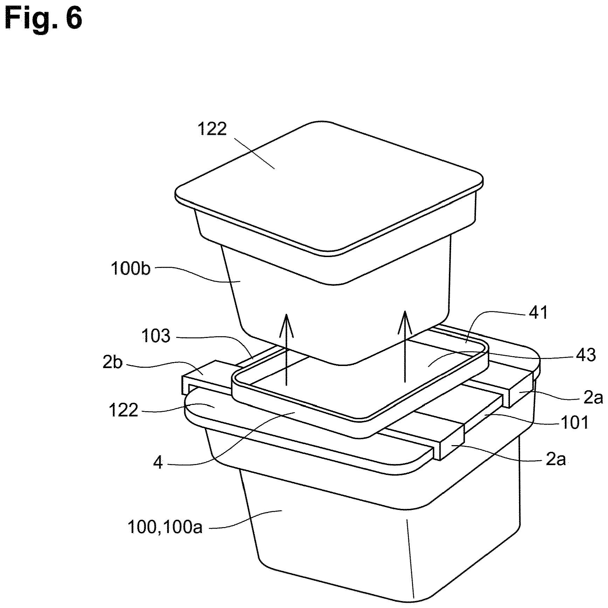

The spacer structure 1 includes a central portion 4 and several supporting arms 2 substantially extending in a horizontal plane orthogonal to the vertical axis A. In the example shown on Figure 4, the spacer structure 1 includes four supporting arms 2 that may form a X-shaped structure. The four supporting arms 2 here engage the four sides of the tubs 100a, 100b. In another embodiment (not shown), the four supporting arms may engage the four corners of the tubs 100a, 100b. In other embodiments (not shown), the spacer structure 1 may include at least three supporting arms 2, or more than four supporting arms 2. If the spacer structure includes three supporting arms 2, these supporting arms 2 may form a Y-shaped structure, for instance two supporting arms 2 engaging two corners of the tubs, and one supporting arm 2 engaging a side of the tubs. With reference to Figure 6, the supporting arms 2 may form a H-shaped structure.

The arms 2 may cross each other substantially at a central portion 4 of the spacer structure 1 and may extend in a straight radial direction from said central portion 4. The central portion 4 extends orthogonally to the vertical axis A and connects an inner end 20 of the supporting arms 2. The central portion 4 is preferably plate-shaped and may be in the form of a square or disc or rectangular plate. The central portion 4 may also be in the form of a ring, such as a rectangular ring as illustrated in figure 6, comprising a window 43 or a notch. Still with reference to Figure 6, the central portion may include a protrusion or rib 41 configured to engage the bottom end 108 of a tub. Preferably, said central portion 4 is as large as the supporting arms 2, as visible in Figure 2. The central portion 4 may however be larger than the supporting arms 2. It is contemplated that the diametrically opposite supporting arms 2 may have the same length.

As visible in Figure 5 or 6, the supporting arms 2 are configured to extend between a cover film 122 of the first (upper) tub 100a and a bottom of the second (lower) tub 100b so as to distribute the load on said cover film (the tubs 100a, 100b are typically transported or stored in an inverted position with regard to the position illustrated in Figure 5 or 6). To that end, the supporting arms 2 may be plate-shaped, thereby defining two opposite plate-shaped first and second sides 24, 26. However, the arms 2 define windows or notches 28 between them, said windows or notches 28 permitting circulation of a sterilization gas by leaving uncovered areas of the cover film.

Although not necessary, adjacent arms 2 may be separated by a predetermined angle and therefore be regularly distributed around the vertical axis A. For example, the four arms 2 may be orthogonal to each other and thus separated by a 90° angle. The angle may be 120° if the spacer structure 1 includes three arms 2 or 45° if the spacer structure 1 includes eight arms 2.

The spacer structure 1 further includes first and second maintaining means for maintaining the first and second tubs 100a, 100b in a stable manner relative to the vertical axis A.

The first maintaining means may be configured to engage the flange 118 of the first tub 100a such that the first tub 100a is laterally blocked by said first maintaining means. They may be provided at the outer end 22 of the supporting arms 2. By outer end it is meant the end furthest from the vertical axis A and the central portion of the spacer structure 1. The inner end, i.e. the end closest to the vertical axis A, of the supporting arms 2 are connected to the central portion 4, and may be connected to each other.

The second maintaining means may be configured to engage the bottom 108 of the second tub 100b such that the second tub 100b is laterally blocked by said second maintaining means. With reference to Figure 4, they may be provided at an intermediate portion of the supporting arms 2 between the outer and inner ends 22, 20 of the supporting arms 2. As shown in Figure 6, the second maintaining means may be provided at the central portion 4, for instance in the form of the protrusion or rib 41; this protrusion or rib 41 may extend circumferentially all around the central portion 4.

The second maintaining extend opposite the first maintaining means. More specifically, the first and second maintaining means are located on opposite sides of the spacer structure 1.

In the illustrative embodiment of Figures 2 and 3, the first and second maintaining means comprise protrusions 6a, 6b, for instance in the form of plate-shaped lugs, that protrude from the respective first and second sides 24, 26 of the supporting arms 2. The first maintaining means may be orthogonal to the supporting arms 2. The second maintaining means may be slightly slanted with regard to the vertical axis A; as visible in Figure 5, they may be configured to extend parallel to the second tub 100b lateral wall 114. For instance, the angle between the second maintaining means and the supporting arms 2 may be around 93°. The first and second maintaining means do not retain, and thereby allow a free vertical movement for fast positioning or removal of the first and second tubs 100a, 100b with respect to the spacer structure 1 and the vertical axis A. In an embodiment (not shown), the spacer structure 1 may be provided with snap fit elements to removably secure the first tub 100a and/or the second tub 100b to the spacer structure 1.

The first and second maintaining means maintain the first and second tubs 100a, 100b aligned and centered along to the vertical axis A.

In the illustrative embodiment of Figures 4 and 6, the first and second maintaining means are configured to engage lateral sides of the first and second tubs 100a, 100b. However, it is contemplated that they may alternatively be configured to engage the corners 120 of the first and second tubs. Accordingly, although not shown in the Figures, the protrusions 6a, 6b may have a corner shape configured to mate the corners 120 of the first or second tub 100a, 100b.

With reference to Figure 4 and 5, each supporting arm 2 is configured to engage a different side of the tub 100a. However, it is possible that several supporting arms 2 engage a same side of the tub 100a, as illustrated in Figure 6 where two supporting arms 2a engage the same side 101 of the tub 100a while two opposite supporting arms 2b engage a same opposite side 103 of the tub 100a.

Preferably, the spacer structure 1 is made of a polymer material or a cellulose material. For instance, the spacer structure 1 may be of polypropylene PP, polystyrene PS, polyethylene terephthalate PET, acrylonitrile-butadiene-styrene ABS, polycarbonate PC, or combinations thereof.

The spacer structure 1, i.e. the central portion 4, the supporting arms 2, and the first and second maintaining means may be made of a single piece.

The Figure 5 shows a packaging 8 including a first tub 100a stacked onto a second tub 100b along a vertical axis A, said first and second tubs 100a, 100b being separated and maintained aligned with respect to the vertical axis A by means of the spacer structure 1. The first side 24 of the spacer structure 1 rests on the cover film of the first tub 100a while the bottom 108 of the second tub 100b rests on the second side 26 of the spacer structure 1.

As visible in Figure 5, the first protrusions 6a engage the flange 118 of the first tub 100a to prevent lateral movements of this first tub 100a while the second protrusions 6b engage the bottom 108 of the second tub 100b to prevent lateral movements of this second tub 100b.

The arms 2 extend between the cover film of the first tub 100a and the bottom wall 116 of the second tub 100b.

Thanks to the spacer structure 1, the stack of the first and second tubs 100a, 100b is stabilized. The cardboard used in order to stack several tubs on a same row is not necessary anymore, and thus it is possible to enclose a line of these tubs 100a, 100b in a single sealing bag 10. It is contemplated that the packaging 8 may comprise more than two stacked tubs 100, such as for instance a stack of three tubs separated by two spacer structures 1 or a stack of four tubs 100 separated by three spacer structures 1.

## Claims

1. A packaging (8) including a second tub (100b) stacked onto a first tub (100a) along a central vertical axis (A), and a spacer structure (1) arranged between the first tub (100a) and the second tub (100b) stacked onto each other along the central vertical axis (A), said first tub (100a) and said second tub (100b) being configured for the transport or storage of a plurality of medical containers (102) such as syringes, wherein the spacer structure (1) comprises:
a central portion (4),
supporting arms (2, 2a, 2b) outwardly extending from said central portion (4), said supporting arms (2, 2a, 2b) and said central portion (4) being configured to be located between a cover film of the first tub (100a) and a bottom (108) of the second tub (100b); wherein the supporting arms (2, 2a, 2b) comprise first maintaining means (6a) configured to prevent radial displacements of the first tub (100a) relative to the spacer structure (1); and
second maintaining means (6b, 41) configured to prevent radial displacements of the second tub (100b) relative to the spacer structure (1).

2. The packaging (8) according to the preceding claim, wherein the supporting arms (2, 2a, 2b) are plate-shaped.

3. The packaging (8) according to any of the preceding claims, wherein the supporting arms (2) are distributed at regular angles around the vertical axis A.

4. The packaging (8) according to any of the preceding claims, wherein the supporting arms (2) form a Y-shaped, H-shaped or X-shaped spacer structure (1).

5. The packaging (8) according to any of the preceding claims, wherein at least two of the supporting arms (2a, 2b) are configured to engage a same side (101, 103) of the first tub (100a).

6. The packaging (8) according to any the preceding claims, wherein the first and the second maintaining means include protrusions (6a, 6b) protruding from, respectively, a first side and an opposite second side of the supporting arms (2).

7. The packaging (8) according to any of the preceding claims, wherein the protrusions (6a, 6b) are shaped to engage lateral sides or corners of, respectively, the first tub (100a) or the second tub (100b).

8. The packaging (8) according to any of the preceding claims, wherein the spacer structure (1) is made of a polymer material, a cellulose material such as molded cellulose or a combination thereof.

9. The packaging (8) according to any of the preceding claims, wherein the central portion comprises a notch or a window (43).

10. The packaging (8) according to any of the preceding claims, wherein the packaging (8) includes a single sealing bag (10) enclosing at least the first tub (100a), the second tub (100b) and the spacer structure (1).

## Patentansprüche

1. Verpackung (8) einschließlich eines zweiten Bechers (100b), der auf einen ersten Becher (100a) entlang einer mittleren vertikalen Achse (A) gestapelt ist, und einer Abstandsstruktur (1), die zwischen dem ersten Becher (100a) und dem zweiten Becher (100b) angeordnet ist, die entlang der mittleren vertikalen Achse (A) aufeinander gestapelt sind, wobei der erste Becher (100a) und der zweite Becher (100b) für den Transport oder die Lagerung einer Vielzahl von medizinischen Containern (102) wie Spritzen konfiguriert sind, wobei die Abstandsstruktur (1) Folgendes umfasst:
einen mittleren Abschnitt (4),
Stützenarme (2, 2a, 2b), die sich von dem mittleren Abschnitt (4) nach außen erstrecken,
wobei die Stützenarme (2, 2a, 2b) und der mittlere Abschnitt (4) so konfiguriert sind, dass sie sich zwischen einem Abdeckungsfilm des ersten Bechers (100a) und einem Boden (108) des zweiten Bechers (100b) befinden;
wobei die Stützenarme (2, 2a, 2b) erste Haltemittel (6a) umfassen, die so konfiguriert sind, dass sie radiale Verschiebungen des ersten Behälters (100a) relativ zu der Abstandsstruktur (1) verhindern; und
zweite Haltemittel (6b, 41), die so konfiguriert sind, dass sie radiale Verschiebungen des zweiten Behälters (100b) relativ zur Abstandsstruktur (1) verhindern.

2. Verpackung (8) nach dem vorstehenden Anspruch, wobei die Stützenarme (2, 2a, 2b) plattenförmig sind.

3. Verpackung (8) nach einem der vorstehenden Ansprüche, wobei die Stützenarme (2) in regelmäßigen Winkeln um die vertikale Achse A verteilt sind.

4. Verpackung (8) nach einem der vorstehenden Ansprüche, wobei die Stützenarme (2) eine Y-förmige, H-förmige oder X-förmige Abstandsstruktur (1) bilden.

5. Verpackung (8) nach einem der vorstehenden Ansprüche, wobei mindestens zwei der Stützenarme (2a, 2b) so konfiguriert sind, dass sie in eine gleiche Seite (101, 103) des ersten Bechers (100a) eingreifen.

6. Verpackung (8) nach einem der vorstehenden Ansprüche, wobei die ersten und zweiten Haltemittel Vorsprünge (6a, 6b) einschließen, die von einer ersten Seite bzw. einer gegenüberliegenden zweiten Seite der Stützenarme (2) vorstehen.

7. Verpackung (8) nach einem der vorstehenden Ansprüche, wobei die Vorsprünge (6a, 6b) so geformt sind, dass sie in die seitlichen Seiten oder Ecken des ersten Bechers (100a) bzw. des zweiten Bechers (100b) eingreifen.

8. Verpackung (8) nach einem der vorstehenden Ansprüche, wobei die Abstandsstruktur (1) aus einem Polymermaterial, einem Zellulosematerial wie beispielsweise geformter Zellulose oder einer Kombination davon hergestellt ist.

9. Verpackung (8) nach einem der vorstehenden Ansprüche, wobei der mittlere Abschnitt eine Kerbe oder ein Fenster (43) umfasst.

10. Verpackung (8) nach einem der vorstehenden Ansprüche, wobei die Verpackung (8) einen einzigen Siegelbeutel (10) einschließt, der mindestens den ersten Becher (100a), den zweiten Becher (100b) und die Abstandsstruktur (1) umschließt.

## Revendications

1. Emballage (8) comprenant un second bac (100b) empilé sur un premier bac (100a) le long d'un axe vertical central (A), et une structure d'espacement (1) agencée entre le premier bac (100a) et le second bac (100b) empilés l'un sur l'autre le long de l'axe vertical central (A),
ledit premier bac (100a) et ledit second bac (100b) étant configurés pour le transport ou le stockage d'une pluralité de récipients médicaux (102) tels que des seringues, dans lequel la structure d'espacement (1) comprend :
une partie centrale (4),
des bras de support (2, 2a, 2b) s'étendant vers l'extérieur depuis ladite partie centrale (4), lesdits bras de support (2, 2a, 2b) et ladite partie centrale (4) étant configurés pour être situés entre un film de recouvrement du premier bac (100a) et un fond (108) du second bac (100b) ; dans lequel les bras de support (2, 2a, 2b) comprennent
des premiers moyens de maintien (6a) configurés pour empêcher les déplacements radiaux du premier bac (100a) par rapport à la structure d'espacement (1) ; et
des seconds moyens de maintien (6b, 41) configurés pour empêcher les déplacements radiaux du second bac (100b) par rapport à la structure d'espacement (1).

2. Emballage (8) selon la revendication précédente, dans lequel les bras de support (2, 2a, 2b) sont en forme de plaque.

3. Emballage (8) selon l'une des revendications précédentes, dans lequel les bras de support (2) sont répartis à angles réguliers autour de l'axe vertical A.

4. Emballage (8) selon l'une des revendications précédentes, dans lequel les bras de support (2) forment une structure d'espacement (1) en forme de Y, en forme de H ou en forme de X.

5. Emballage (8) selon l'une des revendications précédentes, dans lequel au moins deux des bras de support (2a, 2b) sont configurés pour s'engager sur un même côté (101, 103) du premier bac (100a).

6. Emballage (8) selon l'une des revendications précédentes, dans lequel les premier et second moyens de maintien comprennent des saillies (6a, 6b) faisant saillie, respectivement, depuis un premier côté et un second côté opposé des bras de support (2).

7. Emballage (8) selon l'une des revendications précédentes, dans lequel les saillies (6a, 6b) sont formées pour s'engager sur des côtés latéraux ou des coins, respectivement, du premier bac (100a) ou du second bac (100b).

8. Emballage (8) selon l'une des revendications précédentes, dans lequel la structure d'espacement (1) est réalisée en un matériau polymère, un matériau cellulosique tel que la cellulose moulée ou une combinaison de ceux-ci.

9. Emballage (8) selon l'une des revendications précédentes, dans lequel la partie centrale comprend une encoche ou une fenêtre (43).

10. Emballage (8) selon l'une des revendications précédentes, dans lequel l'emballage (8) comprend un seul sac hermétique (10) renfermant au moins le premier bac (100a), le second bac (100b) et la structure d'espacement (100b).
